# EUROPÄISCHE PATENTSCHRIFT

(11) **EP 1 565 225 B1**
(45) Veröffentlichungstag und Bekanntmachung des Hinweises auf die Patenterteilung: **28.04.2010**
(21) Anmeldenummer: 03767435.5
(22) Anmeldetag: 20.11.2003
(51) Int. Cl.: A61M 25/00, A61B 1/273

(54) **MAGENSONDE UND VERFAHREN ZUM EINFÜHREN EINER MAGENSONDE**
GASTRIC TUBE AND METHOD FOR INTRODUCING THE SAME
SONDE GASTRIQUE ET PROCEDE D'INTRODUCTION D'UNE SONDE GASTRIQUE

(30) Priorität: 21.11.2002 DE 10254568
(43) Veröffentlichungstag der Anmeldung: 24.08.2005
(73) Patentinhaber: Westfälische Wilhelms-Universität Münster, 48149 Münster (DE)
(72) Erfinder: Dziewas, Rainer, Westfälische Wilhelms-Univ., 48129 Münster (DE); Perez-Mengual, Salvador, 48129 Münster (DE); Lüdemann, Peter, D-59227 Ahlen (DE)
(74) Vertreter: Viering, Jentschura & Partner
(86) Internationale Anmeldenummer: PCT/DE2003/003835
(87) Internationale Veröffentlichungsnummer: WO 2004/047897

(56) Entgegenhaltungen:
- EP-A- 0 437 229
- EP-A- 0 827 756
- DE-A- 3 307 185
- DE-A- 3 640 034
- DE-C- 19 832 835
- GB-A- 2 059 774
- US-A- 3 858 577
- US-A- 5 846 181
- US-A- 5 989 183
- US-A1- 2002 045 855

## Beschreibung

Die Erfindung betrifft eine Magensonde.

Eine Magensonde dient insbesondere zur Zufuhr bzw. Entnahme von Stoffen in bzw. aus dem Magentrakt eines Patienten. Wenn ein an Schluckstörungen leidender Patient künstlich ernährt werden muss, kann dem Magen des Patienten mittels einer Magensonde Nahrung etwa in Form einer Nährlösung zugeführt werden. Da Schluckstörungen insbesondere durch Bewußtseinsstörungen hervorgerufen werden können, sind Magensonden insbesondere in solchen Fällen für die künstliche Ernährung von großer Bedeutung.

Magensonden werden ferner auch zu Untersuchungszwecken eingesetzt, um den Mageninhalt des Patienten zwecks dessen Untersuchung außerhalb des Körpers vollständig oder teilweise zu entnehmen.

Zum Einführen des dem Magentrakt zuzuführenden Endes der Magensonde wird dieses entweder über Mund (orale Sonde) oder Nase (nasogastrische Sonde) zugeführt und, unterstützt durch einen Schluckvorgang des Patienten, in die Speiseröhre, und, wenn erforderlich oder gewünscht, in den Magen vorgeschoben. Insbesondere bei längerer Verweildauer der Sonde ist die nasogastrische Zufuhr hierzu besonders geeignet.

Eine Magensonde gemäß dem Stand der Technik weist im allgemeinen zwei unabhängige Lumen auf, von denen das eine zur Stoffzufuhr bzw. -abfuhr und das andere zum Druckausgleich mittels Be- oder Entlüftung des Magens dient.

Eine solche Magensonde ist beispielsweise aus US 5,643,230 bekannt und weist einen durch eine Trennwand in zwei unabhängige Lumen (ein größeres Sauglumen zur Entnahme des Mageninhalts und ein kleineres Belüftungslumen zum Druckausgleich) unterteilten Tubus auf. Am offenen extrakorporalen Ende des Tubus ist eine Saugpumpe an das Sauglumen anschließbar, und am dem Magentrakt zuzuführenden Ende des Tubus ist ein flexibles Endstück mit einer Mehrzahl sich longitudinal erstreckender Rippen und hierzwischen angeordneten Saugöffnungen vorgesehen, durch welche der Mageninhalt in das Sauglumen zwecks dessen Entnahme zu Untersuchungszwecken eingesaugt wird.

Aus US 5,560,747 ist es beispielsweise auch bekannt, die nasogastrische Sonde zur Realisierung mehrerer Funktionen (z.B. pH-Analyse etc.) mit einem mehrkanaligen Aufbau auszubilden, wobei der flexible und kompressible Tubus eine Mehrzahl von räumlich voneinander getrennten Durchgangskanälen aufweist. Einer dieser Durchgangskanäle bildet eine innere Führungshülse aus flexiblem, thermoplastischem Material für einen durch ihn hindurchgeführten Nahrungszufuhrtubus.

Beim Vorschub des Magensonde besteht grundsätzlich die Gefahr, dass empfindliches Gewebe infolge Perforation durch die Magensonde verletzt wird. Zur Verringerung der Verletzungsgefahr ist es aus US 5,700,252 bekannt, den aus einem relativ steifen Material hergestellten Tubus der Magensonde mit einer flexiblen Spitze zu versehen. Indem sich die Spitze bei Abweichung von der zentralen Vorschublinie während des Vorschubs der Sonde krümmt, wird durch die Krümmung eine Steuerung des Tubusvorschubs weg von der Gewebewand bewirkt und das Risiko einer Perforation des Gewebes vermindert. Aus US 5,334,167 ist bekannt, den Tubus für die Nahrungszufuhr durch eine an der Magensonde umfangsseitig angebrachte, aus Polyvinylchlorid hergestellte Hülse hindurchzuführen.

DE 198 32 835 C offenbart eine Endosonographievorrichtung, bei welcher ein Einführungsteil eines Endoskops von einem flexiblen Schlauch umhüllt ist. Weiterhin weist das Einführungsteil einen Spülmediumkanal auf, um nach dem Einführen der Vorrichtung in den untersuchten Körperbereich störende Stoffe oder Partikel entfernen zu können. Dieser Spülmediumkanal ist als separater Kanal innerhalb des Einführungsteils ausgebildet, aber nicht an diesem fixiert.

Bei diesen Magensonden kann die Verletzungsgefahr von Patienten mit normalem Bewußtseinszustand bis zu einem gewissen Grade verringert werden, allerdings ist hier oft die Mithilfe des Patienten erforderlich.

Die zuvor beschriebenen Magensonden weisen jedoch den Nachteil auf, dass ein selbständiger Schluckvorgang des Patienten während des Einführens der Magensonde erforderlich ist, was die Zufuhr der Magensonde bis in den Magentrakt bei Patienten mit fehlender oder eingeschränkter Kooperationsbereitschaft oder -fähigkeit erheblich erschwert. Infolgedessen wird während des Einführens der Sonde nicht nur die Belastung des Patienten, sondern auch die Gefahr der Verletzung von empfindlichem Gewebe wesentlich erhöht. Dies ist insbesondere bei Schlaganfallpatienten sowohl in der akuten Erkrankungsphase als auch in der Rehabilitationsphase der Fall, da bei diesen Patienten die Kooperationsfähigkeit durch Beeinträchtigungen der Wachsamkeit und Aufmerksamkeit (sog. Vigilanzminderungen), ein Verlust des Sprachverständnisses (Aphasie) des Patienten durch Schädigungen der zuständigen Hirnabschnitte, und/oder einer Unfähigkeit des Patienten zur Ausführung gezielter Bewegungen (Apraxie) erheblich beeinträchtigt ist.

Es ist bekannt, in diesen Fällen das Einführen der Magensonde durch zusätzliche Maßnahmen wie insbesondere die Sichtkontrolle im Wege der Kehlkopfspiegelung mittels eines Laryngoskops zu erleichtern. Diese zusätzlich eingesetzten Mittel führen jedoch zu einer weiteren Belastung der Patienten sowie zur Erhöhung der Verletzungsgefahr.

Aufgabe der vorliegenden Erfindung ist es daher, eine Magensonde zum Einführen in den Magentrakt eines Patienten zu schaffen, durch die insbesondere auch bei bewußtseinsgestörten Patienten die Belastung des Patienten und die Gefahr von Verletzungen verringert wird.

Diese Aufgabe wird mittels der Magensonde gemäß den Merkmalen des unabhängigen Patentanspruchs gelöst.

Eine Magensonde zur Zufuhr bzw. Entnahme von Stoffen in bzw. aus dem Magentrakt eines Patienten weist ein erstes Schlauchelement auf, welches in den Magentrakt eines Patienten unter Ausbildung eines Förderlumens für dem Magentrakt zuzuführende bzw. zu entnehmende Stoffe oder zur Aufnahme und Einführung von endoskopischen Instrumenten oder chirurgischen Instrumenten geeignet ist. In den Fällen zur Zu- oder Abführung von Stoffen ist das erste Schlauchelement aus einem zumindest teilweise flexiblen Material gebildet, das bevorzugt hautverträglich, insbesondere gegenüber Schleimhäuten ist. Die Magensonde weist ferner ein zweites Schlauchelement auf, welches an dem ersten Schlauchelement fixiert und mit Mitteln zum Auslösen des Schluckreflexes des Patienten versehen ist, wobei das zweite Schlauchelement eine relativ zum ersten Schlauchelement erhöhte Steifigkeit aufweist. Das Auslösen des Schluckreflexes erfolgt vorzugsweise durch physikalischen Reiz der Rachenwand, beispielsweise durch Beaufschlagen mit einer kleinen Menge von Wasser.

Die Fixierung des zweiten Schlauchelements an dem ersten Schlauchelement kann insbesondere eine lösbare Fixierung sein, d.h. es sind vorzugsweise Mittel zur lösbaren Fixierung des zweiten Schlauchelements an dem ersten Schlauchelement vorgesehen. Diese Mittel können beispielsweise einen am extrakorporalen Ende der Sonde zwischen dem ersten und dem zweiten Schlauchelement platzierbaren elastischen Stopfen, z.B. aus einem Kautschukmaterial oder dergleichen, aufweisen, wodurch eine lösbare Verbindung zwischen dem ersten und dem zweiten Schlauchelement hergestellt wird und der nach dem Auslösen des Schluckreflexes und dem Einführen der Magensonde in den Magentrakt ebenso wie das zweite Schlauchelement in einfacher Weise entfernt werden kann.

Alternativ zu der o.g. Fixierung mittels eines elastischen Stopfens können auch beliebige andere Mittel zur lösbaren Fixierung des zweiten Schlauchelements an dem ersten Schlauchelement verwendet werden.

Zur besseren Lösbarkeit des zweiten Schlauchelements nach dem Einführen der Magensonde in den Magentrakt kann das zweite Schlauchelement auch mit einem Gleitmittel, beispielsweise Silikonöl, ummantelt sein.

Die Erfindung ist jedoch auch für Anwendungen einsetzbar, bei denen etwa eine Sonde zur Gastroskopie, Echo-Kardiographie oder Oesophagusskopie durch das Förderlumen hindurchgeführt werden soll. Insbesondere erfolgt im Falle des beabsichtigten Hindurchführens einer Sonde zur Oesophagusskopie durch das Förderlumen kein Vorschub des ersten und zweiten Schlauchelements bis in den Magen, sondern nur bis in die Speiseröhre. Im Sinne der Erfindung umfasst Magentrakt somit die Speiseröhre und den Magen.

Die Erfindung betrifft daher auch eine weitere Ausführung der Magensonde zur Zufuhr von endoskopischen Instrumenten in die Speiseröhre oder Magen eines Patienten, wobei das erste Schlauchelement (101, 201) in Form eines Endoskopieschlauches ausgebildet ist, und vorzugsweise zumindest teilweise mit einem hautverträglichen Material beschichtet ist oder aus diesem gebildet ist.

Bei dieser Ausführungsform ist an einem Endoskopieschlauch, wie er im Stand der Technik bekannt ist, ein zweites Schlauchelement vorgesehen, welches an dem Endoskopieschlauch fixiert oder in diesen integriert ist und mit Mitteln zum Auslösen des Schluckreflexes des Patienten versehen ist. Bei dieser Ausführungsform kann die Fixierung insbesondere auch eine lösbare Fixierung sein.

Infolge des mit Mitteln zum Auslösen des Schluckreflexes versehenen zweiten Schlauchelements kann das Einführen der Magensonde auch bei bewußtseinsgestörten Patienten erfolgen, da zum Einführen der Magensonde bis in den Magen kein vom Patienten bewußt ausgeübter Schluckvorgang erforderlich ist. Infolgedessen ist das Einführen auch bei bewußtseinsgestörten Patienten mit eingeschränkter oder fehlender Kooperationsfähigkeit möglich, ohne daß während des Einführens der Magensonde die Verletzungsgefahr erhöht wird.

Da das erste Schlauchelement, welches zur Ausbildung eines Förderlumens dient und demzufolge einen vergleichsweise großen Durchmesser aufweisen sollte, vorzugsweise aus einem hautverträglichen und flexiblen Material gebildet ist, ist die Gefahr einer Schädigung durch Perforation des empfindlichen Körpergewebes durch dieses Schlauchelement gering.

Zugleich wird durch die erhöhte Steifigkeit des zweiten Schlauchelements, welches lediglich zum Auslösen des Schluckreflexes des Patienten dient und daher nur einen relativ geringen Durchmesser aufzuweisen braucht, eine gute Steuerbarkeit der Magensonde gewährleistet, da das zweite Schlauchelement an dem ersten fixiert ist. Auf diese Weise kann bei sorgfältigem Einführen bis in den Magen problemlos ein ausreichender Abstand zwischen der Magensonde und dem umgebenden Körpergewebe eingehalten werden.

Bevorzugt weisen die Mittel zum Auslösen des Schluckreflexes eine an das zweite Schlauchelement angeschlossene Förderpumpe zum Pumpen einer Flüssigkeit durch das zweite Schlauchelement hindurch auf. Die Förderpumpe ist vorzugsweise eine Dosierpumpe zum Pumpen einer dosierten Flüssigkeitsmenge durch das zweite Schlauchelement hindurch, wobei die Dosiermenge der Förderpumpe insbesondere und vorzugsweise in einem Bereich von (0,5-2,0) ml einstellbar ist. Durch diese definierte Einstellbarkeit ist insbesondere eine gute Reproduzierbarkeit des Einführvorgangs durch Wiederholbarkeit einer optimal eingestellten Dosiermenge gewährleistet.

Vorzugsweise ist der Innendurchmesser des zweiten Schlauchelements durch kapillarartige Ausbildung des zweiten Schlauchelements derart gering gewählt, dass das zweite Schlauchelement infolge seiner Kapillarität vor dem Einführvorgang vollständig mit Flüssigkeit gefüllt ist. Auf diese Weise ist gewährleistet, dass nach dem Einführen des dem Magentrakt zuzuführenden Endes der Magensonde und dem Vorschieben dieses Endes bis auf Höhe der Rachenhinterwand unterhalb der Gaumenbögen keine Luft, sondern unmittelbar die Flüssigkeit zum Auslösen des Schluckreflexes zugeführt wird.

Gemäß einer bevorzugten Ausführungsform ist das zweite Schlauchelement radial innenseitig des ersten Schlauchelements angeordnet. Hierbei können insbesondere das erste und das zweite Schlauchelement zueinander koaxial angeordnet sein. Auf diese Weise ist das zweite Schlauchelement mit größerer Steifigkeit gegenüber dem umgebenden Körpergewebe vollständig durch das erste, weichere und hautverträglichere Schlauchelement abgeschirmt, so dass die Verletzungsgefahr minimiert wird.

Gemäß einer bevorzugten Ausführungsform sind Mittel zum Verhindern eines Herausgleitens des zweiten Schlauchelements aus dem ersten Schlauchelement bei Einführen der Magensonde in den Magentrakt vorgesehen.

Diese Mittel können beispielsweise als ein am dem Magentrakt zuzuführenden Ende der Magensonde innenseitig des ersten Schlauchelements fest angebrachter Gummiring oder auch als eine Verdickung des ersten Schlauchelements an seinem dem Magentrakt zuzuführenden Ende ausgebildet sein.

Des weiteren kann das zweite Schlauchelement auch vorzugsweise kürzer als das erste Schlauchelement ausgebildet sein, so dass das zweite Schlauchelement während des Einführvorganges der Magensonde in den Magentrakt mit dem, dem Magentrakt zuzuführenden Ende nicht aus dem ersten Schlauchelement heraustritt. Vorzugsweise ist hierzu das zweite Schlauchelement relativ zu dem ersten Schlauchelement bis zu der Länge verkürzt, welche etwa dem Außendurchmesser des ersten Schlauchelements entspricht.

Gemäß einer anderen Ausführungsform kann das zweite Schlauchelement auch am Außenrand des ersten Schlauchelements in Längsrichtung parallel zu diesem fixiert sein. Diese Ausführungsform ist insbesondere bei Einführen von endoskopischen und/oder chirurgischen Instrumenten, z.B. Messer zur Entnahme von Gewebeproben im Magentrakt, etc, besonders bevorzugt, da ein sicheres Auslösen des Schluckreflexes so gewährleistet ist und gleichzeitig die Instrumente problemlos eingeführt werden können.

Gemäß einer weiteren bevorzugten Ausführungsform weist das zweite Schlauchelement zur Erhöhung der Steifigkeit relativ zum ersten Schlauchelement eine erhöhte Shorehärte (festgelegt in DIN 53505) auf.

Gemäß einer weiteren bevorzugten Ausführungsform kann das zweite Schlauchelement aus einem Material hergestellt sein, dessen Shorehärte um etwa 10% größer als die Shorehärte des Materials ist, aus dem das erste Schlauchelement hergestellt ist.

Gemäß einer weiteren bevorzugten Ausführungsform kann als Material für das erste Schlauchelement ein thermoplastischer oder elastomerer Kunststoff mit einer Shorehärte A von 70-90, vorzugsweise etwa 80 verwendet werden. Als Material für das zweite Schlauchelement kann beispielsweise ein Kunststoff wie Hart-PVC mit einer Shorehärte D von etwa 40 bis 60 verwendet werden. Vorzugsweise ist das zweite Schlauchelement außerdem aus einem Material gebildet, welches eine so geringe Kompressibilität aufweist, dass während des Einführens der Magensonde, d.h. bei "normalem" Handling derselben keine Flüssigkeit aus dem zweiten Schlauchelement austritt.

Alternativ oder zusätzlich hierzu kann das zweite Schlauchelement zur Erhöhung der Steifigkeit auch relativ zum ersten Schlauchelement eine Mehrzahl von umfangsseitig angeordneten Verstärkungsrippen aufweisen.

Gemäß einer bevorzugten Ausführungsform ist das erste und/oder zweite Schlauchelement aus einem elastomeren Kunststoff hergestellt, wobei der elastomere Kunststoff ein Polykondensat, Polymerisat oder Polyadukt sein kann.

Ferner ist das erste und/oder zweite Schlauchelement bevorzugt aus einem Material hergestellt, welches aus einer Gruppe ausgewählt ist, die Polyurethan, Polyamid, Polyolefin oder Silikon umfasst.

Gemäß einer weiteren bevorzugten Ausführungsform ist das erste und/oder zweite Schlauchelement aus einem Material hergestellt, dem ein Additiv und/oder ein Weichmacher zugesetzt ist.

Gemäß einer weiteren bevorzugten Ausführungsform ist die Elastizität des ersten und/oder zweiten Schlauchelements über den Polymerisationsgrad, den Vernetzungsgrad und/oder den Verzweigungsgrad des verwendeten Kunststoffs gesteuert.

Gemäß einer weiteren bevorzugten Ausführungsform ist das zweite Schlauchelement zur Erhöhung der Steifigkeit aus einem vorzugsweise glasfaserverstärkten Verbundkunststoff hergestellt.

Alternativ oder zusätzlich kann das zweite Schlauchelement zur Erhöhung der Steifigkeit relativ zum ersten Schlauchelement auch eine Mehrzahl von umfangsseitig angeordneten Verstärkungsrippen aufweisen.

Gemäß einer bevorzugten Ausführungsform ist das erste Schlauchelement an seinem dem Magentrakt zuzuführenden Ende abgerundet oder konisch zulaufend ausgebildet, wodurch eine Verletzungsgefahr weiter verringert wird.

Des weiteren kann das erste Schlauchelement auch außenseitig mit Markierungen versehen sein, welche etwa die typischerweise geeignete Einführtiefe der Sonde bis zum Auslösen des Schluckreflexes kennzeichnen.

Zur effektiven Auslösung des Schluckreflexes mit einer verhältnismäßig geringen Dosiermenge weist das zweite Schlauchelement an seinem dem Magentrakt zuzuführenden Endabschnitt einen Düsenaufsatz zum Ausstoß der Flüssigkeitsmenge aus dem zweiten Schlauchelement mit erhöhter Geschwindigkeit auf.

Vorzugsweise besitzt das zweite Schlauchelement einen Außendurchmesser im Bereich von (0,8-1,2) mm, und das erste Schlauchelements besitzt einen Außendurchmesser im Bereich von (3-5) mm.

Gemäß einer weiteren bevorzugten Ausführungsform ist ferner ein drittes Schlauchelement vorgesehen, welches ein Ventilationslumen zur Druckentlastung des Magens bei Förderung über das Förderlumen ausbildet. Auf diese Weise wird bei Zufuhr bzw. Entnahme von Stoffen in bzw. aus dem Magentrakt eines Patienten das Entstehen eines Über- oder Unterdruckes im Magen wirksam verhindert.

Das dritte Schlauchelement kann vorzugsweise ebenfalls radial innenseitig, insbesondere koaxial zu dem ersten Schlauchelement angeordnet sein. Auf diese Weise wird auch das dritte Schlauchelement vollständig durch das erste, weichere und hautverträglichere gegenüber dem umgebenden Körpergewebe abgeschirmt und die Verletzungsgefahr minimiert.

Vorzugsweise ist das dritte Schlauchelement relativ zu dem ersten Schlauchelement in axialer Richtung unter Ausbildung eines über das dem Magentrakt zuzuführende Ende des dritten Schlauchabschnitts vorstehenden Bereichs des ersten Schlauchabschnitts verkürzt. Bei dieser Ausgestaltung wird verhindert, dass sich etwa bei Saugbetrieb des dritten Schlauchelements zur Druckentlastung des Magens, bei Förderung beispielsweise einer Nährstofflösung in den Magen, das dem Magentrakt zuzuführenden Ende des dritten Schlauchelements an der Magenwand festsaugt, da es von dieser durch den vorstehenden Bereich des ersten Schlauchabschnitts beabstandet ist.

Gemäß einer weiteren bevorzugten Ausführungsform weist das erste Schlauchelement an seinem dem Magentrakt zuzuführenden Endabschnitt umfangsseitig angeordnete Luftlöcher auf. Bei dieser Ausgestaltung kann die Ventilation zur Druckentlastung des Mageninneren durch das Ventilationslumen des dritten Schlauchelements durch die Luftlöcher hindurch erfolgen, so daß bei Absaugen von Luft aus dem Mageninneren über das Ventilationslumen zwecks Druckausgleich die Gefahr eines Festsaugens des dem Magentrakt zuzuführenden Endes der Magensonde an der Magenwand weiter verringert wird.

Ein Verfahren zum Einführen der Magensonde zur Zufuhr bzw. Entnahme von Stoffen in bzw. aus dem Magentrakt eines Patienten, weist die Schritte auf:
- orales oder transnasales Einführen eines aus einem hautverträglichen und flexiblen Material gebildeten ersten Schlauchelementes und eines an dem ersten Schlauchelement fixierten sowie eine gegenüber diesem erhöhte Steifigkeit zur Ermöglichung des Vorschubs in den Magentrakt des Patienten aufweisenden zweiten Schlauchelementes bis auf Höhe der Rachenhinterwand unterhalb der Gaumenbögen des Patienten;
- Auslösen des Schluckreflexes des Patienten durch Aufbringen eines vorzugsweise mechanischen Reizes auf die Rachenwand; und
- weiteres Vorschieben des ersten und zweiten Schlauchelementes (101, 201, 108, 208) in die Speiseröhre, und gegebenenfalls in den Magen, derart, dass das zweite Schlauchelement (108, 208) ein Förderlumen (102, 202) für dem Magentrakt zuzuführende bzw. zu entnehmende Stoffe ausbildet.

Zum externen Auslösen des Schluckreflexes des Patienten wird gemäß einer bevorzugten Ausführungsform eine dosierte Flüssigkeitsmenge durch das zweite Schlauchelement hindurch derart gepumpt, dass die dosierte Flüssigkeitsmenge am dem Magentrakt zuzuführenden Ende auf Höhe der Rachenhinterwand unterhalb der Gaumenbögen austritt.

Vorzugsweise ist das zweite Schlauchelement zu Beginn des Einführvorganges vollständig mit Flüssigkeit gefüllt. Als Flüssigkeit kann beispielsweise destilliertes Wasser verwendet werden.

Vorzugsweise werden das erste und das zweite Schlauchelement bis in den Magen des Patienten derart vorgeschoben, daß das Förderlumen die Zufuhr bzw. Entnahme von Stoffen in den Magen des Patienten ermöglicht.

Vorzugsweise wird nach dem Einführen des dem Magentrakt zuzuführenden Endes der Magensonde in den Magen des Patienten ein Druckausgleich im Magen mittels Gasabfuhr über ein von einem dritten Schlauchelement vorgesehenen Ventilationslumen vorgenommen.

Vorzugsweise werden das erste und das zweite Schlauchelement bis in den Magentrakt des Patienten derart vorgeschoben, dass das Förderlumen das Hindurchführen einer Sonde zur Gastroskopie, Echo-Kardiographie oder Oesophagoskopie durch das Förderlumen hindurch ermöglicht.

Die Erfindung wird nachstehend anhand von in den beigefügten Abbildungen dargestellten Ausführungsbeispielen näher erläutert.

Es zeigen:
- Figur 1: eine schematische Seitenansicht einer erfindungsgemäßen Magensonde gemäß einer ersten bevorzugten Ausführungsform der vorliegenden Erfindung;
- Figur 2: eine Querschnittsansicht der Magensonde entlang der Linie I-I aus Figur 1.
- Figur 3: eine schematische Seitenansicht einer erfindungsgemäßen Magensonde gemäß einer zweiten bevorzugten Ausführungsform der vorliegenden Erfindung; und
- Figur 4: eine Querschnittsansicht der Magensonde entlang der Linie III-III aus Figur 3.

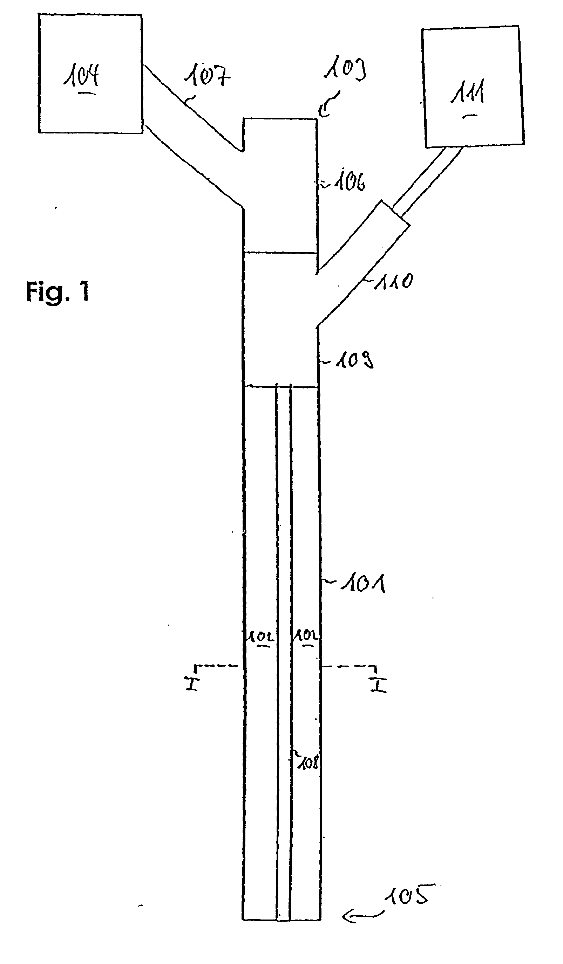

Gemäß Fig. 1 weist eine Magensonde 100 gemäß einem ersten Ausführungsbeispiel ein erstes Schlauchelement 101 auf, welches ein Förderlumen 102 ausbildet. Das erste Schlauchelement 101 ist aus einem hautverträglichen und weichen Material, wie beispielsweise einem elastomeren Kunststoff, hergestellt und weist einen Außendurchmesser von 3-5 mm sowie eine Wanddicke von etwa (0,5-2,0) mm auf.

Das Förderlumen 102 erstreckt sich von einem extrakorporalen Ende 103, wo es an eine Förderpumpe 104 zur Förderung von dem Magentrakt zuzuführenden bzw. zu entnehmenden Stoffen angeschlossen ist, bis zu einem dem Magentrakt zuzuführenden Ende 105 der Magensonde 100, welches bis in den Magen des Patienten vorgeschoben werden kann. Zum Anschluß der Förderpumpe 104 an das extrakorporale Ende 103 der Magensonde 100 ist an dem extrakorporalen Ende 103 ein Verbindungsstück 106 mit einem zu der Förderpumpe 104 führenden Abzweig 107 angebracht.

Radial innenseitig des ersten Schlauchelements 101 sowie koaxial zu diesem ist ein zweites Schlauchelement 108 angeordnet und an dem ersten Schlauchelement 101 fixiert. Das zweite Schlauchelement 108 besitzt einen Außendurchmesser von etwa (0,8-1,2) mm sowie eine Wanddicke von etwa (0,5-1,5) mm und ist über einen benachbart zu dem ersten Verbindungsstück 106 angeordneten zweiten Verbindungsstück 109, der einen Abzweig 110 aufweist, an dem extrakorporalen Ende 106 der Magensonde 100 an eine Dosierpumpe 111 derart angeschlossen, dass eine Flüssigkeit, vorzugsweise destilliertes Wasser, durch das zweite Schlauchelement 108 hindurch bis zum dem Magentrakt zuzuführenden Ende 105 der Magensonde 100 gepumpt werden kann. Die Dosiermenge der Dosierpumpe 111 ist in einem Bereich von (0,5-2,0) ml einstellbar.

Gemäß einer alternativen, jedoch weniger bevorzugten Ausführungsform kann anstelle der Dosierpumpe 111 auch eine Förderpumpe ohne einstellbare Dosiermenge oder auch eine manuell betätigbare Spritze verwendet werden. Ferner können selbstverständlich das Verbindungsstück 106 und das Verbindungsstück 109 auch einstückig als ein einziges Verbindungsstück mit doppeltem Abzweig ausgebildet sein oder die entsprechenden, zu der Förderpumpe 104 bzw. der Dosierpumpe 111 führenden Abzweige können integral mit dem ersten bzw. zweiten Schlauchelement 101, 108 verbunden sein.

Im Betrieb der erfindungsgemäßen Magensonde 100 gemäß dem ersten Ausführungsbeispiel wird das dem Magentrakt zuzuführende Ende 105 der Magensonde 100, d.h. beider aneinander fixierter Schlauchelemente 101 und 108, durch ein Nasenloch des Patienten in die Rachenhöhle vorgeschoben, bis sich das dem Magentrakt zuzuführende Ende 105 etwa auf Höhe der Rachenhinterwand unterhalb der Gaumenbögen befindet, was typischerweise einer Einführlänge von etwa (11-13) cm entspricht. Während dieses Vorschubs ist das zweite Schlauchelement 108 vorzugsweise bereits vollständig mit der durch die Dosierpumpe zugeführten Flüssigkeit (z.B. destilliertes Wasser) angefüllt.

Sobald diese Position erreicht ist, wird über die Dosierpumpe 111 die dort eingestellte Dosiermenge von beispielsweise 0,5 ml destilliertem Wasser in das zweite Schlauchelement 108 eingepumpt, so dass die Flüssigkeit an dem dem Magentrakt zuzuführenden Ende 105 austritt und auf die Rachenhinterwand unterhalb der Gaumenbögen aufgespritzt wird. Hierdurch wird der -visuell problemlos anhand der charakteristischen Kehlkopfbewegung beobachtbare- Schluckreflex des Patienten ausgelöst, woraufhin das dem Magentrakt zuzuführende Ende 105 der Magensonde 100 vorsichtig weiter bis in den Magen des Patienten vorgeschoben wird.

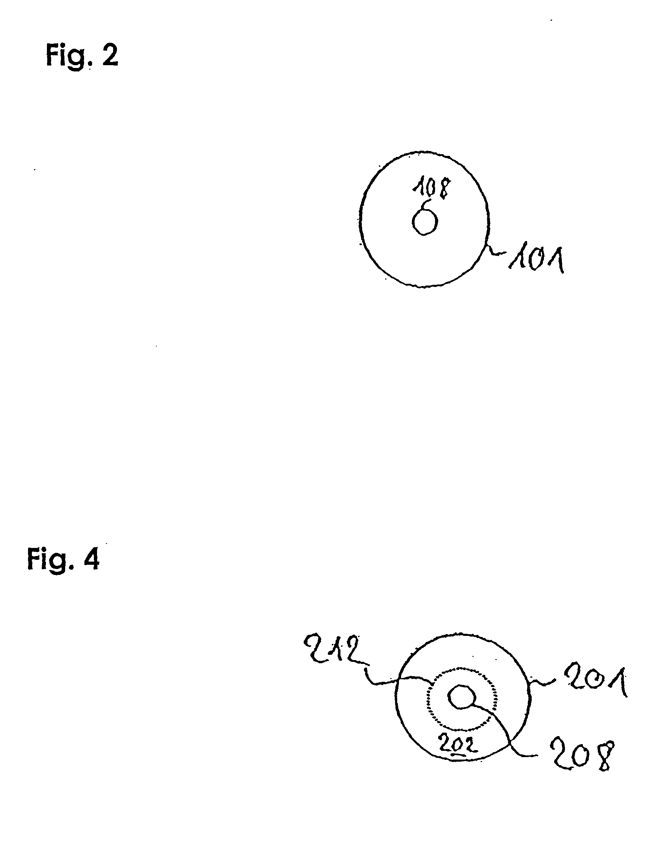

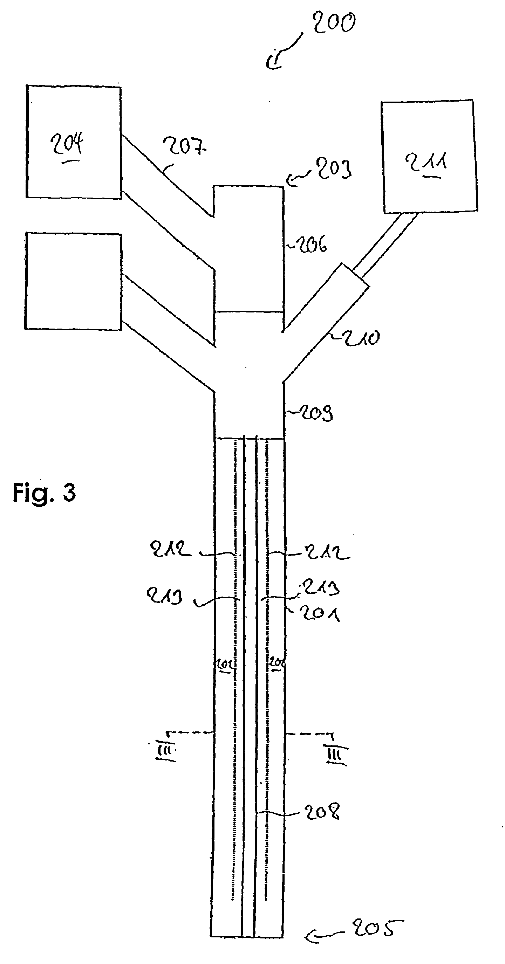

Ein weiteres Ausführungsbeispiel einer erfindungsgemäßen Magensonde 200 ist in Fig. 3 und 4 dargestellt, wobei zu der Magensonde 100 gleiche oder ähnliche Elemente der Magensonde 200 mit entsprechenden Bezugszeichen gekennzeichnet sind, so dass auf deren detaillierte Erläuterung nachfolgend verzichtet wird.

Die Magensonde 200 ist entsprechend der Magensonde 100 aufgebaut, weist jedoch im Unterschied zu der Magensonde 100 zusätzlich ein drittes Schlauchelement 212 auf, welches in radialer Richtung zwischen dem ersten Schlauchelement 201 und dem zweiten Schlauchelement 208 sowie koaxial zu diesen angeordnet ist. Das dritte Schlauchelement 212 bildet ein Ventilationslumen 213 zur Druckentlastung des Magens bei Förderung über das Förderlumen 202 aus, so dass bei Zufuhr bzw. Entnahme von Stoffen in bzw. aus dem Magen des Patienten infolge des Druckausgleichs über das Ventilationslumen das Entstehen eines Über- oder Unterdruckes im Magen wirksam verhindert wird. Hierzu ist das dritte Schlauchelement 212 an seinem extrakorporalen Ende an eine Ventilationspumpe 214 angeschlossen. Dies erfolgt gemäß Fig. 3 über einen weiteren Abzweig 215 des Verbindungsstücks 209, wobei selbstverständlich auch ein hiervon separates Verbindungsstück oder eine integrale Ausbildung mit dem dritten Schlauchelement vorgesehen sein kann.

Wie aus Fig. 3 ersichtlich, ist das dritte Schlauchelement 212 relativ zu dem ersten Schlauchelement 201 in axialer Richtung der Magensonde 200 unter Ausbildung eines über das dem Magentrakt zuzuführende Ende des dritten Schlauchabschnitts 212 vorstehenden Bereichs des ersten Schlauchabschnitts 201 verkürzt. So wird verhindert, dass sich das dem Magentrakt zuzuführende Ende des dritten Schlauchelements 212 an der Magenwand festsaugt, wenn beispielsweise etwa zur Druckentlastung des Magens bei Förderung einer Nährstofflösung Luft aus dem Magen über das Ventilationslumen 213 abgesaugt wird.

Das erste Schlauchelement 201 weist zur Erleichterung des Absaugens bzw. der Zufuhr von Luft über das Ventilationslumen 213 ferner an seinem dem Magentrakt zuzuführenden Endabschnitt umfangsseitig angeordnete Luftlöcher (nicht dargestellt) auf, die vorzugsweise nur in dem oben beschriebenen, über das dem Magentrakt zuzuführenden Ende des dritten Schlauchabschnitts 212 vorstehenden Bereich des ersten Schlauchabschnitts 201 vorgesehen sind. Auf diese Weise wird die Gefahr eines Festsaugens des dem Magentrakt zuzuführenden Endabschnitts der Magensonde weiter reduziert.

Die erfindungsgemäße Magensonde ist vorzugsweise als nasogastrische Sonde ausgebildet. Grundsätzlich möglich, jedoch weniger bevorzugt, ist auch die Ausbildung als orale Sonde, insbesondere falls keine längeren Verweildauern beabsichtigt sind und die Magensonde nur für eine kurzfristige Untersuchung appliziert werden soll.

Das Einführen der erfindungsgemäßen Magensonde kann auch bei bewußtseinsgestörten Patienten mit eingeschränkter oder fehlender Kooperationsfähigkeit erfolgen, ohne daß während des Einführens der Magensonde die Verletzungsgefahr erhöht würde, da zum Einführen der Magensonde bis in den Magen kein vom Patienten bewußt ausgeübter Schluckvorgang erforderlich ist.

Da das erste Schlauchelement aus einem hautverträglichen und flexiblen Material gebildet ist, ist die Gefahr einer Schädigung durch Perforation des empfindlichen Körpergewebes durch dieses Schlauchelement gering. Zugleich wird durch die erhöhte Steifigkeit des zweiten Schlauchelements eine gute Steuerbarkeit der Magensonde gewährleistet. Auf diese Weise kann bei sorgfältigem Einführen bis in den Magen problemlos ein ausreichender Abstand zwischen der Magensonde und dem umgebenden Körpergewebe eingehalten werden, so daß die Verletzungsgefahr auch bei bewußtseinsgestörten Patienten minimiert wird.

### Bezugszeichenliste

- 100: Magensonde
- 101: erstes Schlauchelement
- 102: Förderlumen
- 103: extrakorporales Ende
- 104: Förderpumpe
- 105: dem Magentrakt zuzuführendes Ende
- 106: Verbindungsstück
- 107: Abzweig
- 108: zweites Schlauchelement
- 109: Verbindungsstück
- 110: Abzweig
- 111: Dosierpumpe

- 200: Magensonde
- 201: erstes Schlauchelement
- 202: Förderlumen
- 203: extrakorporales Ende
- 204: Förderpumpe
- 205: dem Magentrakt zuzuführendes Ende
- 206: Verbindungsstück
- 207: Abzweig
- 208: zweites Schlauchelement
- 209: Verbindungsstück
- 210: Abzweig
- 211: Dosierpumpe
- 212: drittes Schlauchelement
- 213: Ventilationslumen
- 214: Ventilationspumpe
- 215: Abzweig

## Patentansprüche

1. Magensonde zur Zufuhr oder Entnahme von Stoffen und/oder endoskopischen und/oder chirurgischen Instrumenten in die/den oder aus der Speiseröhre oder dem Magen eines Patienten,
- mit einem ersten Schlauchelement (101, 201), welches in den Magen eines Patienten unter Ausbildung eines Förderlumens (102) für dem Magentrakt zuzuführende oder zu entnehmende Stoffe oder endoskopische oder chirurgische Instrumente einführbar ist, wobei das erste Schlauchelement (101, 201) zumindest teilweise aus flexiblem Material, das bevorzugt hautverträglich ist, gebildet ist;
- und einem innerhalb des ersten Schlauchelementes (101,102) angeordneten zweiten Schlauchelement (108, 208),
**dadurch gekennzeichnet,**
**dass** das zweite Schlauchelement (108, 208) an dem ersten Schlauchelement fixiert oder in dieses integriert ist und eine relativ zum ersten Schlauchelement (101, 201) erhöhte Steifigkeit aufweist sowie als ein Bestandteil von Mitteln zum Auslösen des Schluckreflexes bei Positionierung seines dem Magentrakt zuzuführenden Endes auf Höhe der Rachenhinterwand unterhalb der Gaumenbögen des Patienten an eine Förderpumpe oder eine Dosierpumpe oder eine Spritze derart angeschlossen oder anschließbar ist, dass zur Auslösung des Schluckreflexes eine dosierte Flüssigkeitsmenge durch das zweite Schlauchelement (108, 208) hindurch dosierbar ist, durch deren Austritt am dem Magentrakt zuzuführenden Ende der Schluckreflex des Patienten auslösbar ist, wobei das zweite Schlauchelement (108, 208) an seinem dem Magentrakt zuzuführenden Endabschnitt einen Düsenaufsatz zum Ausstoß der Flüssigkeitsmenge aus dem zweiten Schlauchelement (108, 208) mit erhöhter Geschwindigkeit aufweist.

2. Magensonde nach Anspruch 1, wobei die zur Auslösung des Schluckreflexes dosierte Flüssigkeitsmenge, die durch das zweite Schlauchelement (108, 208) hindurch dosierbar ist, 0,5 bis 2 ml beträgt.

3. Magensonde nach Anspruch 1 oder 2, wobei die Mittel zum Auslösen des Schluckreflexes eine an das zweite Schlauchelement (108, 208) angeschlossene Förderpumpe zum Pumpen einer Flüssigkeit durch das zweite Schlauchelement (108, 208) hindurch aufweisen.

4. Magensonde nach Anspruch 3, wobei die Förderpumpe eine Dosierpumpe (111,211) zum Pumpen einer dosierten Flüssigkeitsmenge durch das zweite Schlauchelement (108, 208) hindurch ist.

5. Magensonde nach Anspruch 4, wobei die Dosiermenge der Dosierpumpe (111, 211) in einem Bereich von 0,5-2,0 ml einstellbar ist.

6. Magensonde nach Anspruch 1 oder 2, wobei die Mittel zum Auslösen des Schluckreflexes eine an das zweite Schlauchelement (108, 208) angeschlossene manuell betätigbare Spritze umfassen.

7. Magensonde nach einem der vorhergehenden Ansprüche, wobei das erste und/oder zweite Schlauchelement (101, 201, 108, 208) aus einem elastomeren Kunststoff hergestellt ist.

8. Magensonde nach Anspruch 7, wobei der elastomere Kunststoff ein Polykondensat, Polymerisat oder Polyadukt ist.

9. Magensonde nach Anspruch 8, wobei das erste und/oder zweite Schlauchelement (101, 201, 108, 208) aus einem Material hergestellt ist, welches aus eine Gruppe ausgewählt ist, die Polyurethan, Polyamid, Polyolefin oder Silikon umfasst.

10. Magensonde nach einem der Ansprüche 7 bis 9, wobei das erste und/oder zweite Schlauchelement (101, 201, 108, 208) aus einem Material hergestellt ist, dem ein Additiv und/oder ein Weichmacher zugesetzt ist.

11. Magensonde nach einem der Ansprüche 7 bis 10, wobei die Elastizität des ersten und/oder zweiten Schlauchelements (101, 201, 108, 208) über den Polymerisationsgrad, den Vernetzungsgrad und/oder den Verzweigungsgrad des verwendeten Kunststoffs gesteuert ist.

12. Magensonde nach einem der vorhergehenden Ansprüche, wobei das zweite Schlauchelement (108, 208) zur Erhöhung der Steifigkeit relativ zum ersten Schlauchelement (101, 201) eine erhöhte Shorehärte aufweist.

13. Magensonde nach Anspruch 12, wobei das erste Schlauchelement (101, 201) eine Shorehärte A im Bereich von 70 bis 90 und das zweite Schlauchelement (108, 208) eine Shorehärte D im Bereich von 40 bis 60 aufweist.

14. Magensonde nach einem der vorhergehenden Ansprüche, wobei das zweite Schlauchelement (108, 208) zur Erhöhung der Steifigkeit aus einem vorzugsweise glasfaserverstärkten Verbundkunststoff hergestellt ist.

15. Magensonde nach einem der vorhergehenden Ansprüche, wobei das zweite Schlauchelement (108, 208) zur Erhöhung der Steifigkeit relativ zum ersten Schlauchelement (101, 201) eine Mehrzahl von umfangsseitig angeordneten Verstärkungsrippen aufweist.

16. Magensonde nach einem der vorhergehenden Ansprüche, wobei Mittel zum lösbaren Fixieren des zweiten Schlauchelements (108, 208) an dem ersten Schlauchelement (101, 201) vorgesehen sind.

17. Magensonde nach einem der vorhergehenden Ansprüche, wobei das zweite Schlauchelement (108, 208) derart kapillarartig ausgebildet ist, dass das zweite Schlauchelement (108, 208) infolge seiner Kapillarität vor dem Einführvorgang vollständig mit Flüssigkeit gefüllt ist.

18. Magensonde nach einem der vorhergehenden Ansprüche, wobei das zweite Schlauchelement (108, 208) radial innenseitig des ersten Schlauchelements (101, 201) angeordnet ist.

19. Magensonde nach Anspruch 18, wobei das erste und das zweite Schlauchelement (101, 201, 108, 208) zueinander koaxial angeordnet sind.

20. Magensonde nach Anspruch 18 oder 19, wobei Mittel zum Verhindern des Herausgleitens des zweiten Schlauchelementes (108, 208) aus dem ersten Schlauchelement (101, 201) bei Einführen der Magensonde in dem Magentrakt vorgesehen sind.

21. Magensonde nach einem der Ansprüche 1 bis 16, wobei das zweite Schlauchelement (108, 208) am Außenrand des ersten Schlauchelements (101, 201) in Längsrichtung parallel fixiert ist.

22. Magensonde nach einem der vorhergehenden Ansprüche, wobei das zweite Schlauchelement (108, 208) einen Außendurchmesser im Bereich von 0,8-1,2 mm und eine Wanddicke im Bereich von 0,5-1,5 mm aufweist.

23. Magensonde nach einem der vorhergehenden Ansprüche, wobei das erste Schlauchelement (101, 201) einen Außendurchmesser im Bereich von 3-5 mm und eine Wanddicke im Bereich von 0,5-2,0 mm aufweist.

24. Magensonde nach einem der vorhergehenden Ansprüche, wobei das erste Schlauchelement (101, 201) an seinem dem Magentrakt zuzuführenden Ende (105) abgerundet und/oder konisch zulaufend ausgebildet ist.

25. Magensonde nach einem der vorhergehenden Ansprüche, wobei das erste Schlauchelement (101, 201) außenseitig mit wenigstens einer Markierung zur Kennzeichnung der typischen Einführtiefe bis zum Auslösen des Schluckreflexes versehen ist.

26. Magensonde nach einem der vorhergehenden Ansprüche, wobei ferner ein drittes Schlauchelement (212) vorgesehen ist, welches ein Ventilationslumen (213) zur Druckentlastung des Magens bei Förderung über das Förderlumen (202) ausbildet.

27. Magensonde nach Anspruch 26, wobei das dritte Schlauchelement (212) radial innenseitig des ersten Schlauchelements (201) angeordnet ist.

28. Magensonde nach Anspruch 27, wobei das dritte Schlauchelement (212) relativ zu dem ersten Schlauchelement (201) in axialer Richtung unter Ausbildung eines über das dem Magentrakt zuzuführende Ende des dritten Schlauchabschnitts (212) vorstehenden Bereichs des ersten Schlauchabschnitts (201) verkürzt ist.

29. Magensonde nach einem der vorhergehenden Ansprüche, wobei das erste Schlauchelement (201) an seinem dem Magentrakt zuzuführenden Endabschnitt umfangsseitig angeordnete Luftlöcher aufweist.

30. Magensonde nach einem der vorhergehenden Ansprüche, wobei das Förderlumen zur Zufuhr oder Entnahme von Stoffen in den Magen des Patienten ausgebildet ist.

31. Magensonde nach einem der vorhergehenden Ansprüche, wobei das Förderlumen derart ausgebildet ist, dass eine Sonde zur Gastroskopie, Echo-Kardiographie oder Oesophagoskopie durch das Förderlumen hindurchführbar ist.

32. Magensonde nach einem der vorhergehenden Ansprüche,
**dadurch gekennzeichnet,**
**dass** das erste Schlauchelement (101, 201) in Form eines Endoskopieschlauches ausgebildet ist.

33. Magensonde nach einem der vorhergehenden Ansprüche, **dadurch gekennzeichnet, dass** das erste Schlauchelement (101, 201) zumindest teilweise mit einem hautverträglichen Material beschichtet ist.

## Claims

1. Gastric tube for the supply or removal of substances and/or endoscopic and/or surgical instruments into or out of the oesophagus or the stomach of a patient, comprising
- a first tube element (101, 201), which is introducible into the stomach of a patient under formation of a supply lumen (102) for substances to be supplied to and removed from the stomach or endoscopic or surgical instruments, wherein the first tube element (101, 201) is at least partially formed from flexible material, which preferably is skin-compatible; and
- a second tube element (108, 208) arranged within the first tube element (101, 102), **characterized in that**,
the second tube element (108, 208) is fixed to the first tube element or integrated into the first tube element and has an increased rigidity relative to the first tube element (101, 201), said second tubular element as part of means for triggering the swallowing reflex upon positioning of the end to be introduced into the gastric tract at the level of the throat rear wall below the palatine arches being connected or being connectable with a supply pump, a dosing pump or a syringe such that for triggering the swallowing reflex a measured quantity of liquid is dosable through the second tube element (108, 208), wherein the exit of the measured quantity of liquid at the end to be introduced into the gastric tract can trigger the swallowing reflex of the patient, wherein the second tube element (108, 208) has, at its end to be introduced in the gastric tract, a nozzle element for ejecting the quantity of liquid from the second tube element (108, 208) with increased speed.

2. Gastric tube according to claim 1, wherein the dosage quantity of the liquid which is dosable through the second tube element (108, 208) for triggering the swallowing reflex is 0.5 to 2 ml.

3. Gastric tube according to claim 1 or 2, wherein the means for triggering the swallowing reflex comprise a supply pump connected to the second tube element (108, 208) for pumping a liquid through the second tube element (108, 208).

4. Gastric tube according to claim 3, wherein the supply pump is a dosing pump (111, 211) for pumping a measured quantity of liquid through the second tube element (108, 208).

5. Gastric tube according to claim 4, wherein the dosage quantity of the dosing pump (111, 211) is adjustable in a range of 0.5 - 2.0 ml.

6. Gastric tube according to claim 1 or 2, wherein the means for triggering the swallowing reflex comprise a manually operable syringe connected to the second tube element (108, 208).

7. Gastric tube according to one of the preceding claims, wherein the first and/or second tube element (101, 201, 108, 208) is manufactured from an elastomeric synthetic material.

8. Gastric tube according to claim 7, wherein the elastomeric synthetic material is a polycondensate, polymer or polyadduct.

9. Gastric tube according to claim 8, wherein the first and/or second tube element (101, 201, 108, 208) is manufactured from a material which is selected from a group comprising polyurethane, polyamide, polyolefin or silicone.

10. Gastric tube according to any one of claims 7 to 9, wherein the first and/or second tube element (101, 201, 108, 208) are manufactured from material to which an additive and/or softener is added.

11. Gastric tube according to any one of claims 7 to 10, wherein the elasticity of the first and/or second tube element (101, 201, 108, 208) is controlled by way of the degree of polymerization, the degree of cross-linkage and/or the degree of branch-off of the synthetic material used.

12. Gastric tube according to one of the preceding claims, wherein the second tube element (108, 208) has an increased Shore hardness for increasing the rigidity relative to the first tube element (101, 201).

13. Gastric tube according to claim 12, wherein the first tube element (101, 201) has a Shore hardness A in the range of 70 to 90 and the second tube element (108, 208) has a Shore hardness D in the range of 40 to 60.

14. Gastric tube according to one of the preceding claims, wherein the second tube element (108, 208) is manufactured from a preferably glass-fiber-reinforced compound synthetic material for the purpose of increasing the rigidity.

15. Gastric tube according to one of the preceding claims, wherein the second tube element (108, 208) has a plurality of circumferentially arranged reinforcement rips for the purpose of increasing the rigidity relative to the first tube element (101, 201).

16. Gastric tube according to one of the preceding claims, wherein means for the detachable fixing of the second tube element (108, 208) to the first tube element (101, 201) are provided.

17. Gastric tube according to one of the preceding claims, wherein the second tube element (108, 208) is formed as a capillary such that the second tube element (108, 208) due to its capillarity can be completely filled with liquid prior to the introduction process.

18. Gastric tube according to one of the preceding claims, wherein the second tube element (108, 208) is arranged radially on the inside of the first tube element (101, 201).

19. Gastric tube according to claim 18, wherein the first and second tube element (101, 201, 108, 208) are each arranged coaxially to each other.

20. Gastric tube according to claim 18 or 19, wherein means are provided to prevent the second tube element (108, 208) from sliding out of the end of the first tube element (101, 201) during introduction of the gastric tube into the gastric tract.

21. Gastric tube according to any one of claims 1 to 16, wherein the second tube element (108, 208) is fixed parallel in the longitudinal direction at the outer periphery of the first tube element (101, 201).

22. Gastric tube according to one of the preceding claims, wherein the second tube element (108, 208) has an outer diameter in the range of 0.8-1.2 mm and a wall thickness in the range of 0.5-1.5 mm.

23. Gastric tube according to one of the preceding claims, wherein the first tube element (101, 201) has an outer diameter in the range of 3-5 mm and a wall thickness in the range of 0.5-2.0 mm.

24. Gastric tube according to one of the preceding claims, wherein the first tube element (101, 201) is rounded off and/or has a conical shape at its end to be introduced into the gastric tract (105).

25. Gastric tube according to one of the preceding claims, wherein the first tube element (101, 201) is provided with at least one marking on the outside for designating the typical insertion depth up to the triggering of the swallowing reflex.

26. Gastric tube according to one of the preceding claims, further comprising a third tubular element (212), which forms a ventilation lumen (213) for relieving stomach pressure during a supply by way of the supply lumen (202).

27. Gastric tube according to claim 26, wherein the third tube element (212) is arranged radially on the inside of the first tube element (201).

28. Gastric tube according to claim 27, wherein the third tube element (212) is shortened relative to the first tube element (201) in the axial direction under formation of a projecting area of the first tube section (201) over the end of the third tube section (212) to be introduced into the gastric tract.

29. Gastric tube according to one of the preceding claims, wherein the first tube element (201) has air holes arranged on the circumference at its end section to be introduced into the gastric tract.

30. Gastric tube according to one of the preceding claims, wherein the supply lumen is formed for the supply or removal of substances into or out of the stomach of the patient.

31. Gastric tube according to one of the preceding claims, wherein the supply lumen is formed such that a tube for gastroscopy, echocardiography or esophagoscopy can be passed through the supply lumen.

32. Gastric tube according to one of the preceding claims, **characterized in that** the first tube element (101, 201) is formed as an endoscopy tube.

33. Gastric tube according to one of the preceding claims, **characterized in that** the first tube element (101, 201) is at least partially coated with a skin-compatible material.

## Revendications

1. Sonde gastrique destinée à introduire ou retirer des substances et/ou des instruments endoscopiques et/ou chirurgicaux dans ou de l'oesophage ou l'estomac d'un patient, comprenant :
- un premier élément de tuyau (101, 201) qui peut être introduit dans l'estomac d'un patient afin de former une lumière de transport (102) pour les substances ou les instruments endoscopiques ou chirurgicaux à introduire dans ou à retirer du tractus digestif, le premier élément de tuyau (101, 201) étant formé au moins en partie d'un matériau souple qui est de préférence compatible avec la peau ;
- et un deuxième élément de tuyau (108, 208) agencé à l'intérieur du premier élément de tuyau (101, 102),
**caractérisée en ce que** le deuxième élément de tuyau(108, 208) est fixé au premier élément de tuyau ou intégré dans celui-ci et présente une rigidité supérieure à celle du premier élément de tuyau (101, 201) et **en ce que**, en tant que composant de moyens servant à déclencher le réflexe de déglutition lors du positionnement de son extrémité à introduire dans le tube digestif à hauteur de la paroi arrière de la gorge en dessous des piliers du voile du palais du patient, il est ou peut être relié à une pompe d'alimentation ou une pompe de dosage ou une seringue de telle façon que, pour déclencher le réflexe de déglutition, une quantité dosée de liquide peut être dosée à travers le deuxième élément de tuyau (108, 208) et, en sortant par l'extrémité à introduire dans le tube digestif, déclencher le réflexe de déglutition du patient, le deuxième élément de tuyau (108, 208) présentant dans sa partie d'extrémité à introduire dans le tube digestif un embout gicleur pour expulser la quantité de liquide hors du deuxième élément de tuyau (108, 208) à une vitesse élevée.

2. Sonde gastrique selon la revendication 1, dans laquelle la quantité dosée de liquide servant à déclencher le réflexe de déglutition qui peut être dosée par le biais du deuxième élément de tuyau (108, 208) est comprise entre 0,5 et 2 ml.

3. Sonde gastrique selon la revendication 1 ou la revendication 2, dans laquelle les moyens de déclenchement du réflexe de déglutition comprennent une pompe d'alimentation reliée au deuxième élément de tuyau (108, 208) pour pomper un liquide à travers le deuxième élément de tuyau (108, 208).

4. Sonde gastrique selon la revendication 3, dans laquelle la pompe d'alimentation est une pompe de dosage (111, 211) servant à pomper une quantité dosée de liquide à travers le deuxième élément de tuyau (108, 208).

5. Sonde gastrique selon la revendication 4, dans laquelle la quantité dosée par la pompe de dosage (111, 211) peut être ajustée dans une plage de 0,5 à 2,0 ml.

6. Sonde gastrique selon la revendication 1 ou la revendication 2, dans laquelle les moyens de déclenchement du réflexe de déglutition comprennent une seringue actionnable manuellement reliée au deuxième élément de tuyau (108, 208).

7. Sonde gastrique selon l'une des revendications précédentes, dans laquelle le premier et/ou le deuxième élément de tuyau (101, 201, 108, 208) est fabriqué à partir d'une matière plastique élastomère.

8. Sonde gastrique selon la revendication 7, dans laquelle la matière plastique élastomère est un produit de polycondensation, polymérisation ou polyaddition.

9. Sonde gastrique selon la revendication 8, dans laquelle le premier et/ou le deuxième élément de tuyau (101, 201, 108, 208) est fabriqué à partir d'un matériau choisi dans un groupe comprenant le polyuréthane, le polyamide, la polyoléfine ou la silicone.

10. Sonde gastrique selon l'une des revendications 7 à 9, dans laquelle le premier et/ou le deuxième élément de tuyau (101, 201, 108, 208) est fabriqué à partir d'un matériau auquel un additif et/ou un plastifiant est ajouté.

11. Sonde gastrique selon l'une des revendications 7 à 10, dans laquelle l'élasticité du premier et/ou du deuxième élément de tuyau (101, 201, 108, 208) est contrôlée par le degré de polymérisation, le degré de réticulation et/ou le degré de ramification de la matière plastique utilisée.

12. Sonde gastrique selon l'une des revendications précédentes, dans laquelle le deuxième élément de tuyau (108, 208) présente une dureté Shore élevée afin d'augmenter sa rigidité par rapport au premier élément de tuyau (101, 201).

13. Sonde gastrique selon la revendication 12, dans laquelle le premier élément de tuyau (101, 201) présente une dureté Shore A dans la plage de 70 à 90 et le deuxième élément de tuyau (108, 208) une dureté Shore D dans la plage de 40 à 60.

14. Sonde gastrique selon l'une des revendications précédentes, dans laquelle le deuxième élément de tuyau (108, 208) est fabriqué à partir d'un matériau synthétique composite, de préférence renforcé de fibres de verre, afin d'augmenter sa rigidité.

15. Sonde gastrique selon l'une des revendications précédentes, dans laquelle le deuxième élément de tuyau (108, 208) présente une pluralité de nervures de renfort disposées sur sa circonférence afin d'augmenter sa rigidité par rapport au premier élément de tuyau (101, 201).

16. Sonde gastrique selon l'une des revendications précédentes, dans laquelle il est prévu des moyens pour fixer le deuxième élément de tuyau (108, 208) de manière amovible au premier élément de tuyau (101, 201).

17. Sonde gastrique selon l'une des revendications précédentes, dans laquelle le deuxième élément de tuyau (108, 208) a une structure capillaire telle que le deuxième élément de tuyau (108, 208), grâce à sa capillarité, est entièrement rempli de liquide avant le processus d'introduction.

18. Sonde gastrique selon l'une des revendications précédentes, dans laquelle le deuxième élément de tuyau (108, 208) est agencé du côté intérieur dans le sens radial du premier élément de tuyau (101, 201).

19. Sonde gastrique selon la revendication 18, dans laquelle le premier et le deuxième élément de tuyau (101, 201, 108, 208) sont disposés coaxialement l'un par rapport à l'autre.

20. Sonde gastrique selon la revendication 18 ou la revendication 19, dans laquelle il est prévu des moyens pour empêcher le deuxième élément de tuyau (108, 208) de glisser hors du premier élément de tuyau (101, 201) lors de l'introduction de la sonde gastrique dans le tractus digestif.

21. Sonde gastrique selon l'une des revendications 1 à 16, dans laquelle le deuxième élément de tuyau (108, 208) est fixé au bord extérieur du premier élément de tuyau (101, 201) parallèlement à la direction longitudinale.

22. Sonde gastrique selon l'une des revendications précédentes, dans laquelle le deuxième élément de tuyau (108, 208) présente un diamètre extérieur dans la plage de 0,8 à 1,2 mm et une épaisseur de paroi dans la plage de 0,5 à 1,5 mm.

23. Sonde gastrique selon l'une des revendications précédentes, dans laquelle le premier élément de tuyau (101, 201) présente un diamètre extérieur dans la plage de 3 à 5 mm et une épaisseur de paroi dans la plage de 0,5 à 2,0 mm.

24. Sonde gastrique selon l'une des revendications précédentes, dans laquelle le premier élément de tuyau (101, 201) est arrondi et/ou se rétrécit de manière conique au niveau de son extrémité (105) à introduire dans le tractus digestif.

25. Sonde gastrique selon l'une des revendications précédentes, dans laquelle le premier élément de tuyau (101, 201) est muni sur sa face extérieure d'au moins un marquage servant à repérer la profondeur d'introduction typique jusqu'au déclenchement du réflexe de déglutition.

26. Sonde gastrique selon l'une des revendications précédentes, dans laquelle il est prévu en outre un troisième élément de tuyau (212) qui forme une lumière de ventilation (213) servant à évacuer la pression de l'estomac lors du transport par le biais de la lumière de transport (202).

27. Sonde gastrique selon la revendication 26, dans laquelle le troisième élément de tuyau (212) est disposé du côté intérieur dans le sens radial du premier élément de tuyau (201).

28. Sonde gastrique selon la revendication 27, dans laquelle le troisième élément de tuyau (212) est raccourci par rapport au premier élément de tuyau (201) dans la direction axiale afin de former une zone du premier segment de tuyau (201) qui dépasse de l'extrémité du troisième segment de tuyau (212) à introduire dans le tractus digestif.

29. Sonde gastrique selon l'une des revendications précédentes, dans laquelle le premier élément de tuyau (201) présente des trous d'aération agencés sur la circonférence dans sa partie d'extrémité à introduire dans le tractus digestif.

30. Sonde gastrique selon l'une des revendications précédentes, dans laquelle la lumière de transport est formée pour introduire ou retirer des substances dans l'estomac du patient.

31. Sonde gastrique selon l'une des revendications précédentes, dans laquelle la lumière de transport est réalisée de manière à permettre le passage d'une sonde de gastroscopie, d'échocardiographie ou d'oesophagoscopie dans la lumière de transport.

32. Sonde gastrique selon l'une des revendications précédentes, **caractérisée en ce que** le premier élément de tuyau (101, 201) est réalisé sous la forme d'un tube d'endoscopie.

33. Sonde gastrique selon l'une des revendications précédentes, **caractérisée en ce que** le premier élément de tuyau (101, 201) est revêtu au moins en partie d'un matériau compatible avec la peau.
